# EUROPÄISCHE PATENTSCHRIFT

(11) **EP 1 092 397 B1**
(45) Veröffentlichungstag und Bekanntmachung des Hinweises auf die Patenterteilung: **07.12.2005**
(21) Anmeldenummer: 00121631.6
(22) Anmeldetag: 04.10.2000
(51) Int. Cl.: A61B 17/32

(54) **Chirurgisches Instrument**
Surgical Instrument
Instrument Chirurgical

(30) Priorität: 13.10.1999 DE 19949422
(43) Veröffentlichungstag der Anmeldung: 18.04.2001
(73) Patentinhaber: Tontarra Medizintechnik GmbH, 78573 Wurmlingen (DE)
(72) Erfinder: Tontarra, Thomas, 78573 Wurmlingen (DE)
(74) Vertreter: Mammel, Ulrike

(56) Entgegenhaltungen:
- DE-A- 19 748 369
- DE-U- 29 718 969
- US-A- 5 961 531

## Beschreibung

Die Erfindung betrifft ein chirurgisches Instrument gemäß dem Oberbegriff des Anspruchs 1.

Bei diesen chirurgischen Instrumenten, die beispielsweise aus der DE 197 48 369 A1 bekannt sind, handelt es sich um Stanzen, welche eingesetzt werden, um bei chirurgischen Eingriffen Gewebe, Knochen oder dergleichen zu entfernen. Diese Vorrichtungen weisen ein Hauptteil und zumindest ein relativ dazu bewegbares Teil, einen sogenannten Schieber, auf sowie einen Handgriff, der einen mit dem Hauptteil verbundenen feststehenden und einen mit dem bewegbaren verbundenen Teil betätigbaren Griff aufweist. Durch Öffnen und Schließen des Griffes wird der bewegbare Teil zum Hauptteil geschlossen und geöffnet, wobei während der Schließbewegung beispielsweise das Entfernen von Gewebe, Knochen oder dergleichen ermöglicht ist.

Zur Reinigung und Sterilisation wird das bewegbare Teil von dem Hauptteil vollständig gelöst und entfernt. Das Hauptteil und das bewegbare Teil sind dabei jeweils mit Kennzeichnungen versehen, so daß nach dem Reinigen und Sterilisieren der Instrumente eine Zuordnung des bewegbaren Teils zum Hauptteil ermöglicht ist. Dies ist erforderlich, da die chirurgischen Instrumente im zusammengebauten Zustand durch Schleifen endbearbeitet werden, um einen absatzfreien Übergang zwischen dem Hauptteil und dem bewegbaren Teil zu ermöglichen. Dies hat zur Folge, daß bei mehreren zu reinigenden Instrumenten eine zeitaufwendige Zuordnung des bewegbaren Teils zum Hauptteil erforderlich ist.

Aus der US-A-5 961 531 ist ein chirurgisches Instrument bekannt, bei dem das bewegbare Teil durch ein relativ zum feststehenden Teil des Handgriffes betätigbares Griffteil in eine Arbeits- und eine Ausgangsposition anordenbar ist. Zum Überführen des bewegbaren Teils aus der Arbeitsposition in eine Reinigungsposition ist erforderlich, dass nach dem Lösen einer Verriegelungsvorrichtung in Form eines Drehknopfes der betätigbare Griff gegenüber dem feststehenden Handgriff mit einer Verschiebebewegung beaufschlagt wird, damit ein Stift des bewegbaren Teils aus einem randoffenen Schlitz des betätigbaren Griffes freikommt, um anschließend das bewegbare Teil in eine Reinigungsposition überzuführen. Das bewegbare Teil wird über eine zusätzliche Lasche zum Handgriff gehalten. Der betätigbare Griff muss somit nach dem Entriegeln der Verriegelungsvorrichtung aktiv bewegt werden, damit ein Stift des bewegbaren Teils aus dem randoffenen Schlitz freikommen kann, um das bewegbare Teil aus einer Ausgangsposition in eine Reinigungsposition zu verschieben.

Diese Vorrichtung weist den Nachteil auf, dass die Handhabung mehrere nacheinander folgende Schritte erfordert, um ein Lösen des bewegbaren Teils von dem Hauptteil zu ermöglichen. Darüber hinaus ist diese Vorrichtung in der Herstellung aufwendig und kostenintensiv.

Der Erfindung liegt deshalb die Aufgabe zugrunde, ein chirurgisches Instrument zu schaffen, welches für die Reinigung und Sterilisation zerlegbar ist, eine Zuordnung der Teile nicht erforderlich ist und in der Handhabung einfach ausgestaltet ist.

Diese Aufgabe wird erfindungsgemäß durch die Merkmale des Anspruchs 1 gelöst.

Durch die erfindungsgemäße Anordnung einer Verriegelungsvorrichtung und der unverlierbaren Ausgestaltung des bewegbaren Teils zum Hauptteil kann ermöglicht werden, daß das chirurgische Instrument für die Reinigung und Sterilisation zumindest hinsichtlich des Hauptteils und bewegbaren Teils in einfacher Wiese getrennt zueinander anordenbar sind, wobei das chirurgische Instrument als eine Einheit erhalten bleibt. Dadurch kann ein aufwendiger Zuordnungsvorgang nach der Reinigung und Sterilisation verhindert werden. Des weiteren kann ein chirurgisches Instrument geschaffen werden, welches die hygienischen Anforderungen erfüllt, da die Gelenkverbindung zwischen dem betätigbaren Griff und dem bewegbaren Teil für die Reinigung gut zugänglich ist. Darüber hinaus ist durch die Ausgestaltung der Verriegelungsvorrichtung ein chirurgisches Instrument geschaffen, welches in der Handhabung den bisherigen Instrumenten entspricht, so daß keine Umgewöhnung für den Operateur erforderlich ist. Des weiteren ist eine einfache Handhabung für die Überführung des beweglichen Teils zum Hauptteil in eine Reinigungsposition ermöglicht. Derartige chirurgische Instrumente können für sämtliche medizinische Bereiche einsetzbar sein. Die erfindungsgemäße Ausgestaltung kann für sämtliche chirurgische Instrumente vorgesehen sein, bei denen ein Hauptteil und ein bewegbares Teil vorgesehen ist und für die Reinigung zumindest teilweise zerlegbar vorzusehen sind.

Nach einer vorteilhaften Ausgestaltung der Erfindung ist vorgesehen, dass das bewegbare Teil und der betätigbare Griff durch eine Gelenkverbindung zueinander schwenkbar angeordnet sind. Dadurch kann vorteilhafterweise die für die Betätigung des bewegbaren Teils bereits vorhandene Gelenkverbindung gleichzeitig für die Anordnung des bewegbaren Teils in einer Reinigungsposition zum Hauptteil verwendet werden. Alternativ kann vorgesehen sein, dass anstelle der gelenk-, kulissen- oder scharnierartigen Gelenkverbindung weitere Verbindungsmechanismen vorgesehen sein können, welche das bewegbare Teil zum Hauptteil unverlierbar anordnen. Dabei kann auch vorgesehen sein, dass die Gelenkverbindung zwischen dem betätigbaren Griff und dem bewegbaren Teil gelöst werden kann und eine weitere Verbindung zur Sicherung des bewegbaren Teils vorgesehen ist.

Nach einer weiteren vorteilhaften Ausgestaltung der Erfindung ist vorgesehen, dass beim Überführen des bewegten Teils aus einer Reinigungsposition in eine Arbeitsposition die Führungsabschnitte des bewegbaren Teils in Aufnahme des Hauptteils eingreift und durch Drücken des betätigbaren Griffs die Führungen selbsttätig ineinander greifen. Dadurch kann ein sicherer und zwangsweiser Zusammenbau erzielt werden, wobei das Drücken des betätigbaren Griffes soweit erfolgt, dass die Verriegelungsvorrichtung selbständig in die Verriegelungsposition oder manuell in diese überführbar ist, so dass im Anschluss daran der Arbeitshub für das bewegbare Teil freigegeben ist.

Nach einer weiteren vorteilhaften Ausgestaltung der Erfindung ist vorgesehen, daß die Führung im Hauptteil einen ersten schrägverlaufenden Abschnitt aufweist, welcher das bewegbare Teil auf das Hauptteil zubewegt und in eine Arbeitsposition überführt. Dadurch kann ein leichtes Zusammenführen und vollständiges Aneinanderliegen der Führungsflächen zwischen dem Hauptteil und bewegbaren Teil erzielt werden.

Nach einer weiteren vorteilhaften Ausgestaltung der Erfindung ist vorgesehen, daß der Riegel kraft- und/oder formschlüssig in einer ersten Verriegelungsposition anordenbar ist. Dadurch kann sichergestellt sein, daß während eines chirurgischen Eingriffes ein selbständiges Öffnen der Verriegelungsvorrichtung verhindert werden kann.

Nach einer vorteilhaften Ausgestaltung der Erfindung ist vorgesehen, daß die Verriegelungsvorrichtung einen Riegel aufweist, der an dem Handgriff vorgesehen ist. Dadurch kann eine einfache Einhandbetätigung für das Entriegeln der Verriegelungsvorrichtung vorgesehen sein. Der Riegel kann sowohl an dem feststehenden Handgriff als auch an dem betätigbaren Griff vorgesehen sein. Diese Anordnung kann in Abhängigkeit der Ausführungsform frei gewählt werden.

Nach einer weiteren vorteilhaften Ausgestaltung ist vorgesehen, daß in dem feststehenden Griff ein schwenkbarer Riegel vorgesehen ist, der nahe einer Schwenkachse an dem betätigbaren Griff angreift. Dadurch kann eine kompakte Bauweise erzielt werden, wobei vorteilhafterweise auf die bisherige Geometrie und Größe der chirurgischen Instrumente zurückgegriffen werden kann.

Nach einer weiteren vorteilhaften Ausgestaltung der Erfindung ist vorgesehen, daß der schwenkbare Riegel einen zu seiner Verriegelungsposition bestimmenden Anschlag aufweist. Dadurch kann der Arbeitshub des bewegbaren Teils begrenzt werden, wobei die Hubbegrenzung des bewegbaren Teils durch den Riegel zumeist die Ausgangsposition des bewegbaren Teils für einen Arbeitshub bestimmt.

Nach einer weiteren vorteilhaften Ausgestaltung der Erfindung ist vorgesehen, daß der Riegel in eine Entriegelungsposition schwenkbar ist und einen weiteren Schwenkbereich des betätigbaren Griffs freigibt, welcher vorzugsweise durch ein an dem Hauptteil vorgesehenen Anschlag begrenzt ist. Dadurch kann der bewegbare Teil relativ zum Hauptteil derart bewegt werden, daß die ineinandergreifenden Führungsabschnitte voneinander getrennt werden können, um das bewegbare Teil um die Gelenkverbindung zu schwenken und von dem Hauptteil zumindest teilweise abzuheben. Die Größe des Schwenkbereiches kann vorteilhafterweise in Abhängigkeit der Länge der ineinandergreifenden Führungen abgestimmt sein, so daß auch ein sehr kurzer Schwenkbereich genügen würde, um die ineinandergreifenden Führungen zwischen dem Hauptteil und dem bewegbaren Teil voneinander zu trennen.

Nach einer weiteren vorteilhaften Ausgestaltung der Erfindung ist vorgesehen, daß der Riegel an einem Abschnitt zwischen einer Schwenkachse des betätigbaren Handgriffs und der Gelenkverbindung angreift. Dadurch kann eine kompakte Bauweise geschaffen werden und der Riegel nahezu vollständig in dem feststehenden Handgriff integriert sein. Darüber hinaus ist diese Verriegelungsvorrichtung für die sonstige Handhabung nicht störend.

Nach einer weiteren vorteilhaften Ausgestaltung der Erfindung ist vorgesehen, daß der Riegel einen Verriegelungsabschnitt aufweist, der an einem komplementär ausgebildeten Schwenkabschnitt des betätigbaren Handgriffs angreift. Dadurch kann eine definierte Anlage des Schwenkabschnitts an dem Verriegelungsabschnitt gegeben sein. Vorteilhafterweise kann dadurch erzielt werden, daß durch die Ausbildung einer Art Hinterschneidung beziehungsweise einer Anlagefläche und einer sich anschließenden Rastnocke erzielt werden kann, daß nach jedem Arbeitshub bei Rückstellung des betätigbaren Griffs in eine Ausgangsposition der Riegel in eine Verriegelungsposition gedrückt wird. Sollte sich der Riegel aus seiner Verriegelungsposition auch nur geringfügig gelöst haben, wird dieser nach jeden Arbeitshub automatisch in diese wieder zurückgeführt. Dadurch kann eine sichere Handhabung gewährleistet sein. Die Anordnung des Riegels an dem feststehenden Handgriff hat des weiteren den Vorteil, daß zwischen dem feststehenden Handgriff und dem Hauptteil ein Übergangsbereich vorgesehen ist, der den Verriegelungsmechanismus nahezu vollständig abschließt, so daß diese Verriegelungsvorrichtung vor mechanischen Beschädigungen von außen gleichzeitig geschützt sein kann.

Weitere vorteilhafte Ausgestaltungen und Weiterbildungen der Erfindung sind in den weiteren Ansprüchen näher beschrieben.

Anhand der nachfolgenden Zeichnungen sind vorteilhafte Ausführungsformen näher beschrieben. Es zeigen:
- Figur 1: eine schematische Seitenansicht einer erfindungsgemäßen Ausführungsform in einer Ausgangsposition,
- Figur 2: eine schematische Seitenansicht der Ausführungsform gemäß Figur 1 in einer Arbeitsposition,
- Figur 3: eine schematische Seitenansicht der Ausführungsform gemäß Figur 1 in einer Zwischenposition mit einer entriegelten Verriegelungsvorrichtung,
- Figur 4: eine schematische Seitenansicht der Ausführungsform gemäß Figur 1 in einer Reinigungsposition,
- Figur 5: eine schematische Seitenansicht einer erfindungsgemäßen Ausführungsform mit einer alternativen Verriegelungsvorrichtung,
- Figur 6: eine schematische Seitenansicht einer erfindungsgemäßen Ausführungsform mit einer weiteren alternativen Verriegelungsvorrichtung und
- Figur 7: eine schematische Seitenansicht einer erfindungsgemäßen Ausführungsform mit einer weiteren alternativen Verriegelungsvorrichtung.

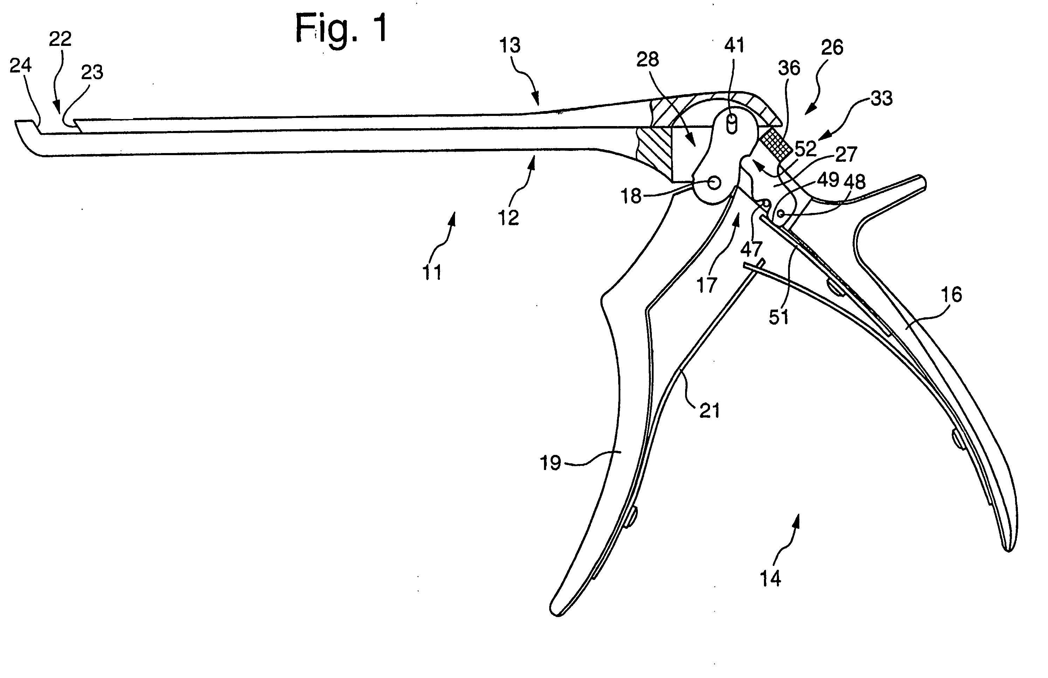

Eine erste erfindungsgemäße Ausführungsform eines chirurgischen Instrumentes 11 ist in den Figuren 1 bis 4 dargestellt. Bei diesem chirurgischen Instrument 11 handelt es sich beispielsweise um eine sogenannte Stanze, welche bei chirurgischen Eingriffen für die Entfernung von Gewebe, Knochen oder dergleichen eingesetzt wird. Die Erfindung ist nicht auf diese Stanzen beschränkt, sondern kann auf alle chirurgischen Instrumente übertragen werden, welche dieselbe Problematik hinsichtlich der Reinigung und Sterilisation sowie Zuordnung von Bauteilen aufweist.

Das chirurgische Instrument gemäß Figur 1 weist einen Hauptteil 12 auf, welches ein bewegbares Teil 13, das relativ zum Hauptteil 12 verschiebbar ist, aufnimmt.

An dem Hauptteil 12 ist ein Handgriff 14 angeordnet. Das Hauptteil 12 geht in einen feststehenden Griff 16 des Handgriffs 14 über und weist im Übergangsbereich 17 vom Hauptteil zum feststehenden Griff 16 eine Drehachse 18 auf, um welche ein betätigbarer Griff 19 schwenkbar angeordnet ist. Der betätigbare Griff 19 und feststehende Griff 16 werden durch ein Federelement 21 in einer Ausgangsposition 22 angeordnet. In dieser Ausgangsposition 22 sind die Schneidelemente 23, 24 zueinander beabstandet, wobei dieser Abstand durch den maximalen Weg eines Arbeitshubes bestimmt ist. Diese Ausgangsposition 22 ist des weiteren durch eine Verriegelungsvorrichtung 26 bestimmt. Ein Riegel 27 der Verriegelungsvorrichtung 26 begrenzt die durch das Federelement 21 bewirkte Schwenkbewegung des betätigbaren Griffs 19 um die Drehachse 18. Dabei liegt ein kurzer Hebelabschnitt 28 an dem Riegel 27 an.

Bei Betätigung des Griffes 19 wird dieser um die Drehachse 18 geschwenkt, wobei das bewegbare Teil 13 gemäß der Darstellung nach links bewegt wird, um das Schneidelement 23 auf das Schneidelement 24 zuzubewegen.

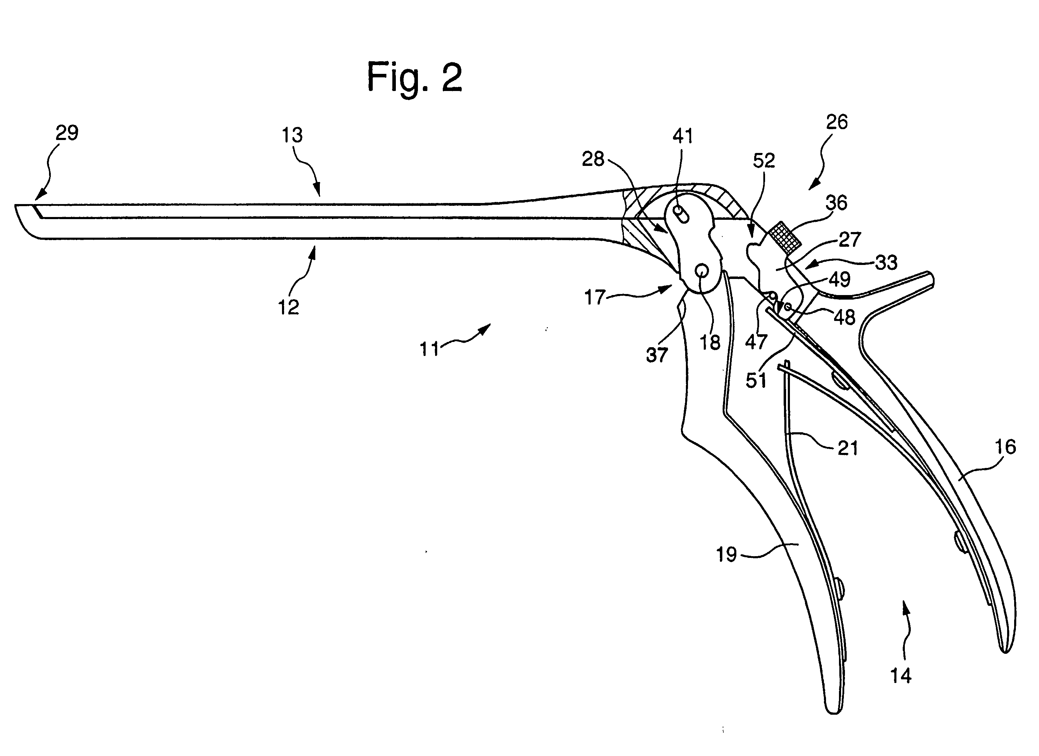

In Figur 2 ist der Arbeitshub beendet und das chirurgische Instrument 11 in eine Arbeitsposition 29 dargestellt. Das zu entfernende Gewebe oder der Knochen oder dergleichen ist in einem innerhalb den Schneidelementen 23, 24 vorgesehenen Hohlraum eingeschlossen und kann somit entfernt werden. Nachdem das chirurgische Instrument 11 aus dem Bereich des chirurgischen Eingriffs entfernt wurde, kann der Handgriff 14 gelöst werden, wodurch das chirurgische Instrument 11 sich selbständig durch die Federelemente 21 in einer Ausgangsposition 22 positioniert. Das zu entfernende Material kann sich dann selbständig von der Schneide 23 lösen, da vorteilhafterweise die Führungselemente 31, 32 als Auswerfer dienen.

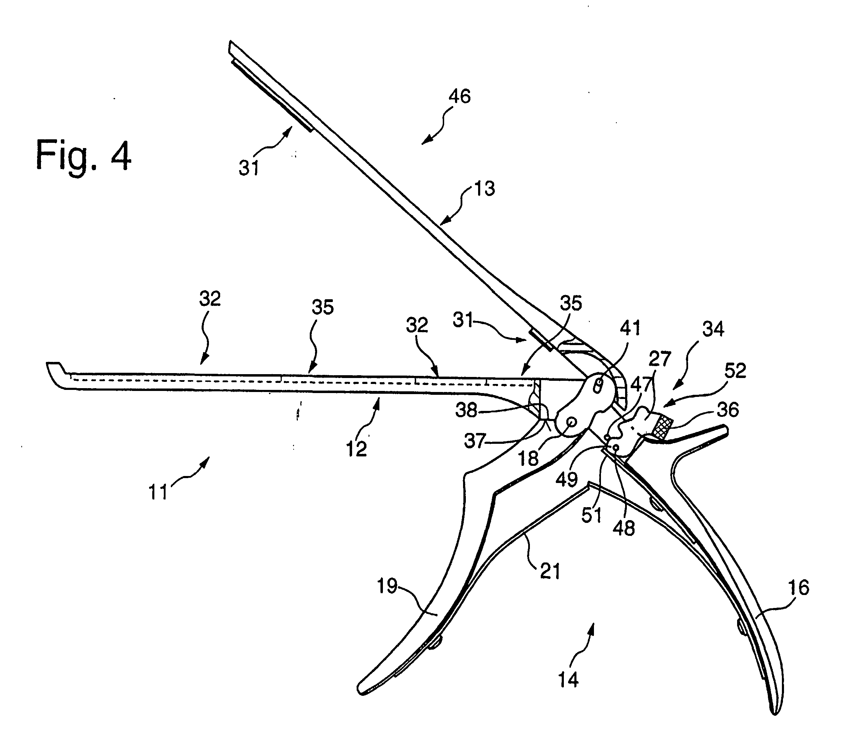

In Figur 4 ist beispielsweise das Führungselement 31 an dem beweglichen Teil 13 dargestellt. Diese vorteilhafterweise T-förmig ausgebildete Feder greift in eine entsprechende Nut, die das Führungselement 32 bildet, im Hauptteil 12 ein. In Abhängigkeit der Ausbildung des chirurgischen Instruments 11 können auch die Führungselemente 31, 32 angepaßt und ausgebildet sein.

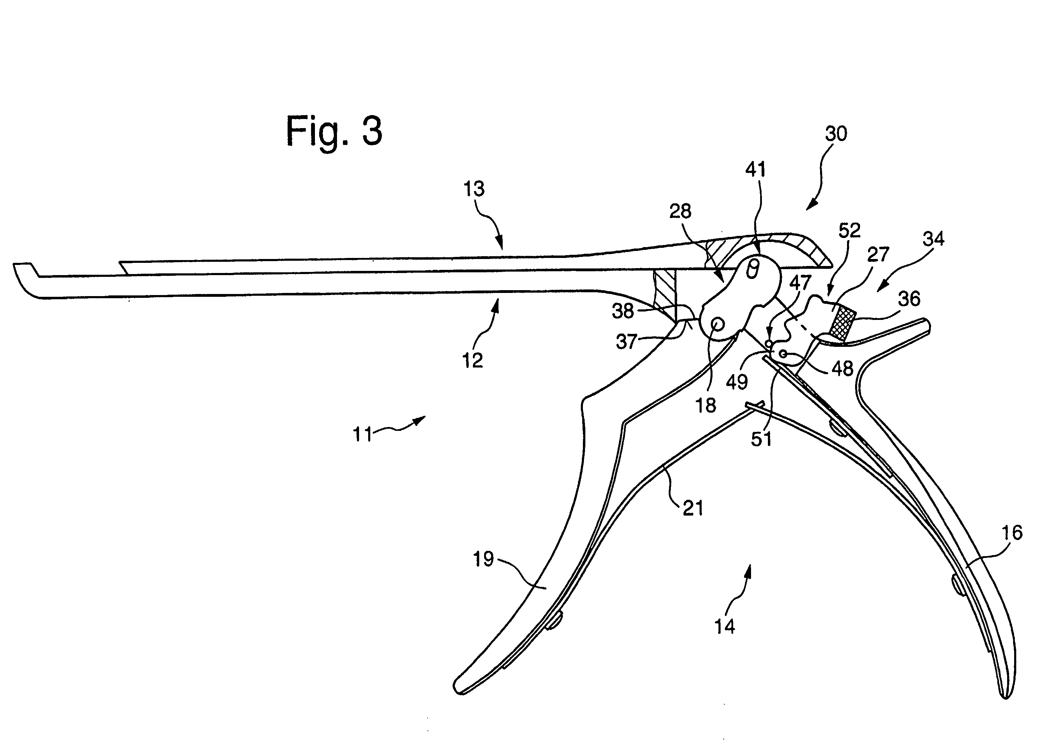

Zur Reinigung und Desinfektion des chirurgischen Instrumentes 11 ist erforderlich, daß das bewegbare Teil 13 von dem Hauptteil 12 zumindest teilweise abgehoben wird, um auch die Zwischenräume zu reinigen und zu desinfizieren. Die Verriegelungsvorrichtung 26 wird aus ihrer Verriegelungsposition 33, wie dies in Figur 1 und 2 dargestellt ist, in eine Entriegelungsposition 34 gemäß Figur 3 und 4 übergeführt. Gemäß der Ausführungsform in den Figuren 1 bis 4 erfolgt dies durch Schwenken des Riegels 27, wobei vorteilhafterweise der betätigbare Handgriff 19 zumindest teilweise auf den feststehenden Handgriff 16 zubewegt ist, so daß der Riegel 7 mittels der Grifffläche 36 in die Entriegelungsposition 34 geschwenkt werden kann. Dadurch wird für den bewegbaren Teil 13 eine weitere Wegstrecke freigegeben, so daß dieser gegenüber dem Hauptteil 12 derart verschiebbar ist, daß die Führungselemente 31, 32 sich gegenseitig freigeben. Dieser Verschiebeweg wird durch einen an dem betätigbaren Handgriff 19 vorgesehenen Anschlag 37 begrenzt, der mit einer entsprechenden Fläche 38 im Übergangsbereich 17 zusammenwirkt.

Der bewegbare Teil 13 und der betätigbare Griff 19 sind vorteilhafterweise über ein Gelenk 41 wirkungsverbunden. Dieses Gelenk 41 ist als schwenkbares Gelenk ausgebildet, welches gleichzeitig eine Längsbewegung der Schwenkachse ermöglicht, um die kreisbogenförmige Bewegung des Hebelabschnittes 28 um die Drehachse 18 in eine Längsbewegung des bewegbaren Teils 13 entlang dem Hauptteil 12 zu ermöglichen.

In Figur 3 ist eine Zwischenposition 30 dargestellt, welche beim Überführen des chirurgischen Instrumentes aus einer Ausgangsposition 22 gemäß Figur 1 in eine Reinigungsposition 46 gemäß Figur 4 durchlaufen wird. Nach dem Entriegeln des Riegels 27 wird das bewegbare Teil 13 in die Zwischenposition 30 gemäß Figur 3 übergeführt, um anschließend durch Schwenken des bewegbaren Teils 13 um das Gelenk 41 in die Reinigungsposition gemäß Figur 4 überzuführen. Dies kann durch einfaches Betätigen oder Drücken des Endabschnittes nach der Verbindung 41 erfolgen, um das bewegbare Teil 13 von dem Hauptteil 12 abzuheben. Diese Anordnung weist den Vorteil auf, daß die Zwischenräume zwischen dem bewegbaren Teil 13 und dem Hauptteil 12 gut zugänglich sind und darüber hinaus die Bauteile des chirurgischen Elementes miteinander gekoppelt sind.

Die Herstellung der Einsatzbereitschaft des chirurgischen Instrumentes 11 erfolgt in umgekehrter Reihenfolge. Zunächst wird das bewegbare Teil 13 auf das Hauptteil 12 zubewegt, wobei die Führungselemente 31 in eine Aufnahme 35 des Hauptteils 12 angreifen, welche dann in die Führungselemente 32 übergehen. Anschließend wird der betätigbare Griff 19 auf den feststehenden Griff 16 zubewegt, wodurch die Führungselemente 31 in die Führungselemente 32 eingreifen. Vorteilhafterweise ist ein erster Abschnitt der Führungselemente 32 schräg angeordnet, so daß das bewegbare Teil während der axialen Bewegung in Richtung Schneide 24 gleichzeitig nach unten auf das Hauptteil 12 zubewegt wird, so daß nahezu ein spaltfreier Übergang zwischen dem bewegbaren Teil 13 und dem Hauptteil 12 in der Ausgangsposition vorgesehen ist. Der Riegel 27 wird in seine Verriegelungsposition 33 übergeführt, wobei dieser an einem Begrenzungselement 47 anliegt. Dadurch kann der Riegel 27 in eine definierte Position übergeführt werden, welche gleichzeitig die Ausgangsposition 22 bestimmt, da das Federelement 21 den betätigbaren Handgriff um die Drehachse 18 bewegt, wodurch der Hebelabschnitt 28 an dem Riegel 27 anliegt.

Der Riegel 27 ist um eine Achse 48 schwenkbar angeordnet. Nahe der Achse 48 ist eine Nase 49 vorgesehen, welche mit einer Feder 51 zusammenwirkt. Diese Feder 51 bewirkt zum einen, daß während der Betätigung des chirurgischen Instrumentes 11 der Riegel 27 in seiner Verriegelungsposition 33 als auch während der Reinigung in einer Entriegelungsposition 34 gehalten wird. Die Nase 49 ist dementsprechend ausgebildet, daß beide Endpositionen fixierbar sind.

Der Riegel 27 weist des weiteren einen Anlageabschnitt 52 auf, der sich aus einem geradlinigen Abschnitt und einem halbkreisförmigen Abschnitt zusammensetzt. Diese Ausgestaltung kann variabel ausgebildet sein, wobei die Funktion füllt sein muß, wonach bei der Bewegung des betätigbaren Griffes 19 aus der Arbeitsposition 29 in eine Ausgangsposition 22 der Hebelabschnitt 28 derart auf den Riegel 27 einwirkt, daß dieser gegen das Begrenzungselement 47 bewegt wird. Dadurch kann sichergestellt werden, daß nach jeden Arbeitshub das Verriegelungselement bei gegebenenfalls sich teilweise lösenden wieder in die Verriegelungsposition 33 zurückgeführt wird.

Alternativ zur Ausführungsform gemäß den Figuren 1 und 4 kann vorgesehen sein, daß der feststehende Griff 16 und der betätigbare Griff 19 vertauscht sind. In Analogie kann hierzu auch der Verriegelungsmechanismus angeordnet sein.

Ebenso ist auch denkbar, daß anstelle eines Riegels 27, der die Ausgangsposition 22 aufgrund einer Druckbelastung hält, diese auch durch eine Zugbelastung hält, beispielsweise dann, wenn das Verriegelungselement spiegelbildlich zu einer Linie angeordnet ist, welche beispielsweise zwischen der Drehachse 18 und dem Gelenk 41 gebildet wäre.

Alternativ kann auch vorgesehen sein, daß anstelle einer Gelenkverbindung, wie dies in den Figuren 1 bis 4 dargestellt ist, eine U-förmige Öffnung oder dergleichen vorgesehen ist, wodurch ein Bolzen oder ein Stift, welcher an dem bewegbaren Teil 13 angeordnet ist, in der Führung im Hebelabschnitt 28 des betätigbaren Griffs 19 läuft. Zusätzlich kann um das bewegbare Teil 13 unverlierbar zum Hauptteil 12 oder zum Handgriff 14 anzuordnen, ein weiteres Sicherungselement, wie beispielsweise eine Leine, eine Kette oder eine weitere stabförmige Gelenkverbindung vorgesehen sein, um einerseits zerlegbar für die Reinigung und Desinfektion zu sein und andererseits das bewegbare Teil 13 unverlierbar zu den weiteren Komponenten des chirurgischen Instruments anzuordnen.

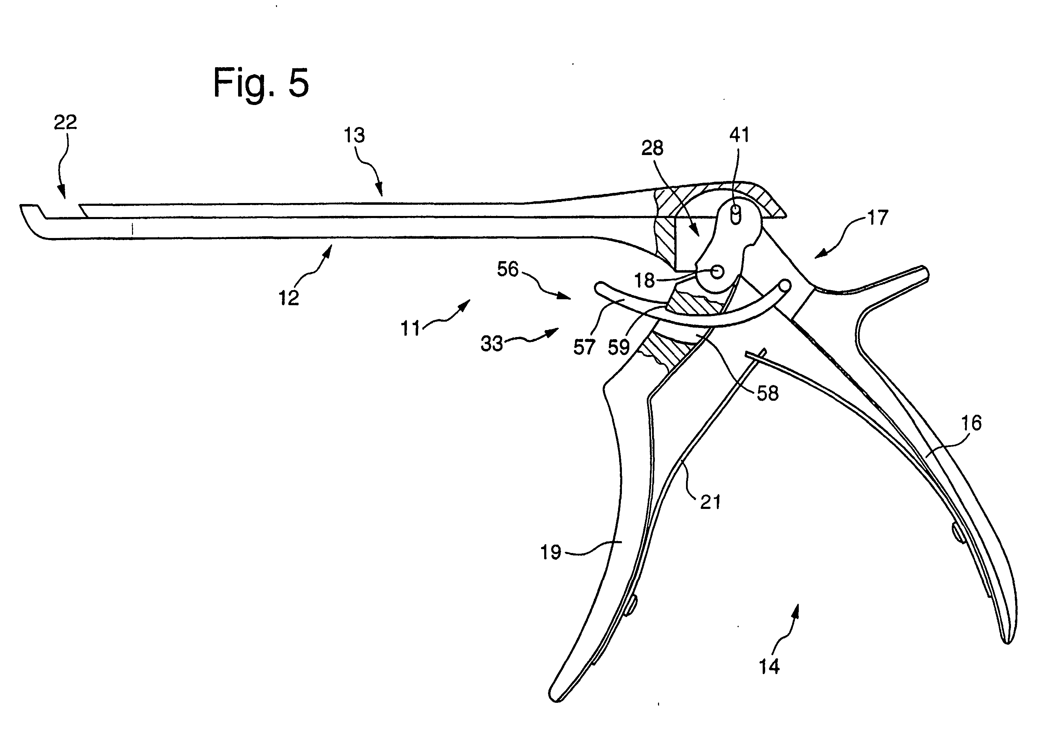

In Figur 5 ist eine alternative Ausführungsform einer Verriegelungsvorrichtung 56 dargestellt. Ein Riegel 57 ist schwenkbar an dem feststehenden Handgriff 16 angeordnet und kreuzt den betätigbaren Griff 19. Dabei ist vorteilhafterweise vorgesehen, daß in dem Griff 19 eine Ausnehmung 58 vorgesehen ist, in welcher der Riegel 57 geführt ist. Dieser weist einen Absatz 59 auf, durch welchen der bewegbare Teil 13 zum Hauptteil 12 in einer Ausgangsposition 22 anordenbar ist. Durch Drücken des Riegels 57 kann dieser Verriegelung gelöst werden, so daß für den Griff 19 ein weiterer Schwenkbereich freigegeben wird, um das bewegbare Teil 13 in eine Zwischenposition 30 gemäß Figur 3 überzuführen, welche anschließend in eine Position 46 gemäß Figur 4 geschwenkt werden kann. Der erweiterte Schwenkbereich des Griffes 19 kann einerseits durch einen Anschlag 37 an einer Fläche 38 des Hauptteils begrenzt sein oder durch eine weitere Rastnase, welche an dem Riegel 57 vorgesehen ist. Der Riegel 57 wird durch eine nicht näher dargestellte Feder in Richtung auf die Drehachse 18 gedrückt oder gezogen und ist während der Betätigung des chirurgischen Instruments 11 nachgiebig, so daß der Handgriff 19 schwenkbar zum feststehenden Griff 16 vorgesehen ist.

Alternativ kann vorgesehen sein, daß die Rastnase auch an einem unteren Abschnitt der Ausnehmung 58 angreift. Um das chirurgische Instrument 11 von einer Ausgangsposition 22 in eine Reinigungsposition 46 überzuführen, ist hierbei erforderlich, daß der Riegel 57 nach oben bewegt wird. Es versteht sich, daß diese Anordnung auch spiegelbildlich vorgesehen sein kann, sowohl hinsichtlich der Anordnung des Riegels als auch hinsichtlich der Griffe 16, 19. Dies gilt auch für die weiteren Ausführungsformen.

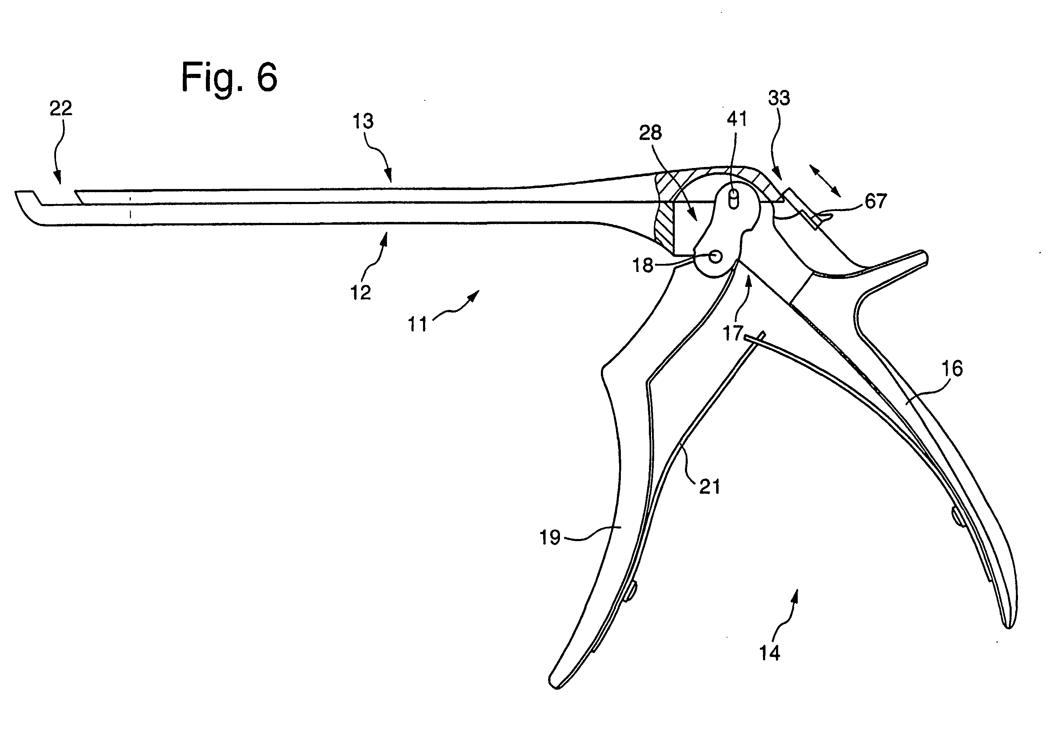

In Figur 6 ist eine weitere alternative Verriegelungsvorrichtung 66 dargestellt. Diese Verriegelungsvorrichtung 66 weist einen Riegel 67 auf, der an einem hinteren Ende des bewegbaren Teil 13 angreift und die Bewegung entlang dem Hauptteil 12 begrenzt. Durch eine Schiebebewegung zum freien Ende des Griffes 16 oder durch eine Schwenkbewegung um eine Drehachse, welche in jede Richtung denkbar ist, kann der Riegel 67 aus einer Verriegelungsposition 33, wie in Figur 6 dargestellt ist, in eine nicht näher dargestellte Entriegelungsposition übergeführt werden. Dadurch kann das bewegbare Teil 13 aus der in Figur 6 dargestellten beitsposition 22 in die in Figur 3 dargestellte Zwischenposition 30 übergeführt werden. Ebenso kann vorgesehen sein, daß der Riegel 67 anstelle eines gemäß der Zeichnung dargestellten Anschlages auch unmittelbar an dem Hebelabschnitt 28, beispielsweise nahe des Gelenkes 41, angreift. Der Riegel 67 kann ebenso an dem bewegten Teil 13 vorgesehen sein und mit dem Handgriff 14 oder dem Hauptteil 12 zusammenwirken.

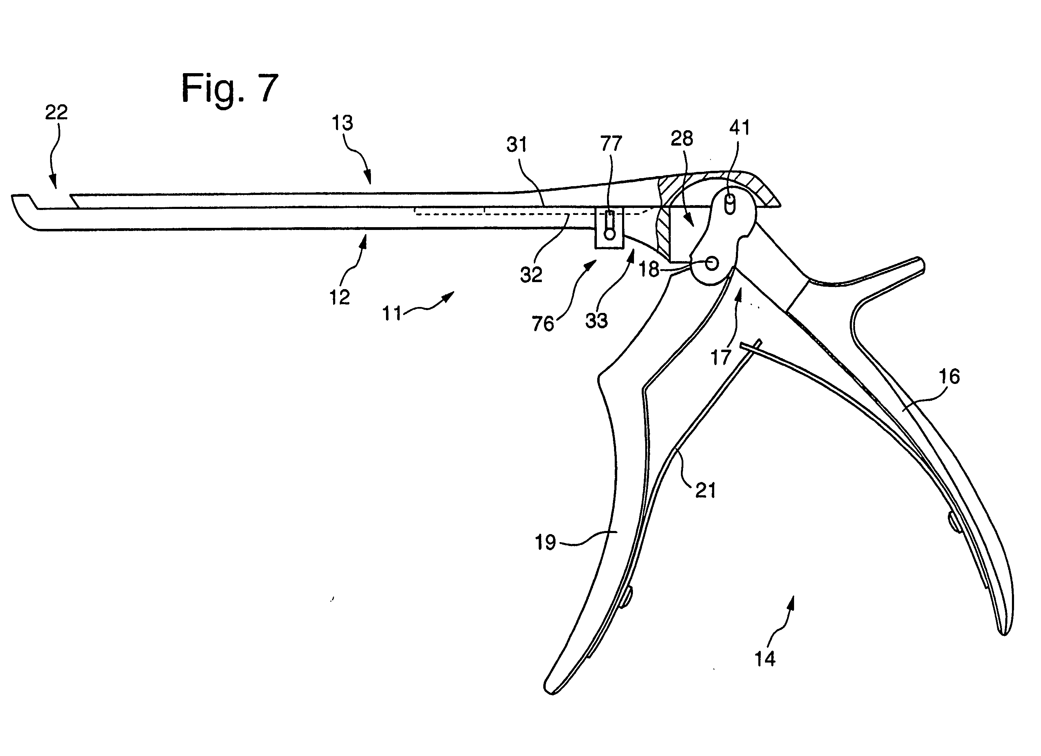

In Figur 7 ist eine weitere alternative Ausführungsform einer Verriegelungsvorrichtung 76 dargestellt. Diese Verriegelungsvorrichtung 76 ist an dem Hauptteil 12 vorgesehen und greift in das Führungselement 32 oder eine Aufnahme 35 ein. In einer Verriegelungsposition 33 sperrt diese Verriegelungsvorrichtung 76 durch einen Riegel 77 die Bewegung des bewegbaren Teiles 13 nach rechts im Sinne der Darstellung beziehungsweise in eine Ausgangsposition 22. Durch Drücken, Ziehen, Schieben oder Klappen um eine Drehachse kann der Riegel 77 betätigt werden und das Führungselement 32 freigeben, so daß das Führungselement 31 aus dem Führungselement 32 herausgeführt werden kann.

Sämtliche Ausführungsformen haben gemeinsam, daß das bewegliche Teil 13 gegenüber dem Hauptteil 12 bewegbar und abnehmbar anordenbar sind, wobei über eine Verbindung das bewegbare Teil zum Hauptteil oder Handgriff unverlierbar vorgesehen sind. Die Verriegelungsvorrichtung kann in Abhängigkeit der Ausführungsform an dem feststehenden Griff 16, dem betätigbaren Griff 19, dem Hauptteil 12 oder dem bewegbaren Teil 13 vorgesehen sein und an zumindest jeweils einem benachbarten Teil oder Griff in einer Verriegelungsposition angreifen. Die Verriegelungsvorrichtung kann durch Ziehen, Schieben, Drücken, Klappen, Schwenken oder dergleichen eines Riegels entriegelt und verriegelt werden. Es versteht sich, daß die entsprechenden für chirurgische Instrumente geeigneten Materialien eingesetzt werden.

Alternativ zu den in den Figuren 1 bis 7 dargestellten chirurgischen Elementen, welche als sogenannte oben-schneidende Stanzen ausgebildet sind, können auch unten-schneidende Stanzen vorgesehen sein. Die Schneiden 23, 24 sind spiegelbildlich zur Führungsebene des beweglichen Teils 13 am Hauptteil 12 vorgesehen. Diese Ausführungsformen können ebenso erfindungsgemäß ausgebildet sein. Das bewegbare Teil 13 ist dabei stufenförmig ausgebildet, wobei ein erster Abschnitt im Bereich der Verbindung 41 beibehalten ist und ein zur Schneide 23 führender Abschnitt auf der Unterseite des Hauptteils 12 verläuft. Damit dieselbe erfindungsgemäße Ausgestaltung ermöglicht und die daraus resultierenden Vorteile erzielt werden können, ist vorgesehen, daß in dem stufenförmigen Übergangsbereich von der Oberseite zur Unterseite ein Gelenk vorgesehen ist, wodurch der vordere stufenförmig abgesetzte Abschnitt bei einer Positionierung des bewegbaren Teils in einer Zwischenstation gemäß Figur 3 gegenüber dem Hauptteil 12 wegschwenkbar ist, um anschließend gemäß Figur 4 schwenkbar zu sein. Derartige Abwandlungen oder ergänzende Maßnahmen, um von den erfindungsgemäßen Vorteilen Gebrauch zu machen, sind ebenfalls erfindungsgemäß umfaßt.

## Patentansprüche

1. Chirurgisches Instrument mit einem Hauptteil (12) und zumindest einem relativ dazu, in einer Führung (31, 32) bewegbaren Teil (13), mit einem an dem Hauptteil (12) angeordneten Handgriff (14), der einen feststehenden Griff (16) und einen das bewegbare Teil (13) betätigbaren Griff (19) aufweist, mit einer Verriegelungsvorrichtung (26, 56, 66, 76), bei der in einer ersten Position (33) das bewegbare Teil (13) in einer Ausgangsposition (22) angeordnet ist und in einer zweiten Position (34) das bewegbare Teil (13) in eine Reinigungsposition (46) überführbar ist und das bewegbare Teil (13) in dieser Reinigungsposition (46) unverlierbar zum Hauptteil (12) angeordnet ist, **dadurch gekennzeichnet, dass** das bewegbare Teil (13) bei Überführung aus der Ausgangsposition (22) in die Reinigungsposition (46) aus der Führung (31, 32) freikommt und um eine Drehachse einer zwischen dem betätigbaren Griff (19) und dem bewegbaren Teil (13) gebildeten Gelenkverbindung (41) zumindest teilweise schwenkbar ist.

2. Chirurgisches Instrument nach Anspruch 1, **dadurch gekennzeichnet, dass** das bewegbare Teil (13) und der betätigbare Griff (19) durch eine gelenk-, kulissen- oder scharnierartige Gelenkverbindung (41) zueinander schwenkbar angeordnet sind.

3. Chirurgisches Instrument nach einem der vorhergehenden Ansprüche, **dadurch gekennzeichnet, dass** beim Überführen des bewegten Teils (13) aus einer Reinigungsposition (46) in die Arbeitsposition (22) die Führungsabschnitte (31) des bewegbaren Teils in Aufnahmen des Hauptteils (12) eingreifen und durch Drücken des betätigbaren Griffs (19) die Führungen (31, 32) selbsttätig ineinander greifen.

4. Chirurgisches Instrument nach Anspruch 3, **dadurch gekennzeichnet, dass** die Führung (32) im Hauptteil (12) einen ersten schrägverlaufenden Abschnitt aufweist, welcher das bewegbare Teil (13) auf das Hauptteil (12) zubewegt und in eine Arbeitsposition (22) überführt.

5. Chirurgisches Instrument nach einen der vorhergehenden Ansprüche, **dadurch gekennzeichnet, dass** die Verriegelungsvorrichtung (26, 56, 67, 77) einen Riegel (27, 57, 67, 77) aufweist, der kraft- und/oder formschlüssig zumindest in einer Verriegelungsposition (33) anordenbar ist.

6. Chirurgisches Instrument nach einem der vorhergehenden Ansprüche, **dadurch gekennzeichnet, dass** die Verriegelungsvorrichtung (26, 56, 66) einen Riegel (27, 57, 67) aufweist, der an dem Handgriff (14) vorgesehen ist.

7. Chirurgisches Instrument nach Anspruch 6, **dadurch gekennzeichnet, dass** der Riegel (27, 57, 67) an dem feststehenden oder betätigbaren Griff (16, 19) vorgesehen ist.

8. Chirurgisches Instrument nach Anspruch 7, **dadurch gekennzeichnet, dass** an dem feststehenden Griff (19) ein schwenkbarer Riegel (27) vorgesehen ist, der nahe einer Drehachse (18) an dem betätigbaren Griff (19) angreift.

9. Chirurgisches Instrument nach Anspruch 8, **dadurch gekennzeichnet, dass** der schwenkbare Riegel (27) einen seine Verriegelungsposition (33) bestimmendes Begrenzungselement (47) aufweist.

10. Chirurgisches Instrument nach einem der Ansprüche 5 bis 9, **dadurch gekennzeichnet, dass** der Riegel (27) in einer Entriegelungsposition (34) schwenkbar einen weiteren Schwenkbereich des betätigbaren Griffs (19) freigibt, welcher von einem an dem Hauptteil (12) vorgesehenen Anschlag (37) begrenzt ist.

11. Chirurgisches Instrument nach einem der Ansprüche 5 bis 10, **dadurch gekennzeichnet, dass** der Riegel (27) an einem Hebelabschnitt (28) zwischen der Schwenkachse (18) und dem Gelenk (41) angreift.

12. Chirurgisches Instrument nach einem der Ansprüche 5 bis 11, **dadurch gekennzeichnet, dass** der Riegel (27) einen Verriegelungsabschnitt aufweist, der an einem komplementär ausgebildeten Hebelabschnitt (28) des betätigbaren Griffs (19) angreift.

13. Chirurgisches Instrument nach Anspruch 12, **dadurch gekennzeichnet, dass** der Verriegelungsabschnitt eine Hinterschneidung aufweist, die vorzugsweise durch eine Schräge und ein sich daran anschließenden Rastnocken ausgebildet ist.

14. Chirurgisches Instrument nach einem der Ansprüche 1 bis 7, **dadurch gekennzeichnet, dass** der Riegel (57) an einem Hebelabschnitt (28) angreift, welcher dem betätigbaren Griff (19) gegenüberliegt.

15. Chirurgisches Instrument nach Anspruch 14, **dadurch gekennzeichnet, dass** der Riegel (57) In einer Ausnehmung (58) des betätigbaren Griffs (19) geführt ist.

16. Chirurgisches Instrument nach einem der Ansprüche 14 oder 15, **dadurch gekennzeichnet, dass** an dem Riegel (57) ein Absatz (59) vorgesehen ist, der den betätigbaren Griff (19) in einer Ausgangsposition (22) positioniert.

17. Chirurgisches Instrument nach einem der Ansprüche 14 bis 16, **dadurch gekennzeichnet, dass** durch Lösen des Riegels (57) von einem Randbereich der Ausnehmung (58) ein weiterer Schwenkbereich des betätigbaren Griffs (19) freigegeben wird und der betätigbare Griff (19) bis zum Anschlag (37) am Hauptteil (12) oder einem weiteren Absatz des Riegels (57) schwenkbar ist.

18. Chirurgisches Instrument nach einem der Ansprüche 14 bis 17, **dadurch gekennzeichnet, dass** beim Überführen des betätigbaren Griffs (19) aus einer Reinigungsposition (46) in eine Arbeitsposition (22) eine selbständige Verriegelung des Riegels (57) vorgesehen ist und vorzugsweise der betätigbare Griff (19) in einer Ausgangsposition angeordnet ist.

19. Chirurgisches Instrument nach einem der Ansprüche 1 bis 7, **dadurch gekennzeichnet, dass** am feststehenden Griff (16) ein Riegel (67) vorgesehen ist, der an dem beweglichen Teil (13), vorzugsweise an einem Endabschnitt des beweglichen Teils (13), angreift.

20. Chirurgisches Instrument nach Anspruch 19, **dadurch gekennzeichnet, dass** der Riegel (67) schwenkbar oder verschiebbar ausgebildet ist.

21. Chirurgisches Element nach einem der Ansprüche 19 oder 20, **dadurch gekennzeichnet, dass** der Riegel (67) in eine Verriegelungsposition (33), vorzugsweise durch eine lösbare Rastverbindung, gesichert ist.

22. Chirurgisches Instrument nach einem der Ansprüche 1 bis 7, **dadurch gekennzeichnet, dass** die Verriegelungsvorrichtung (67) einen Riegel (77) aufweist, der an dem Hauptteil (12) oder bewegbaren Teil (13) vorgesehen ist.

23. Chirurgisches Instrument nach Anspruch 22, **dadurch gekennzeichnet, dass** die an dem Hauptteil (12) vorgesehene Verriegelungsvorrichtung (76) einen Riegel (77) aufweist, der in eine Führung (32) im Hauptteil (12) eingreift, in welcher der bewegbare Teil (13) zumindest abschnittsweise geführt ist.

24. Chirurgisches Instrument nach einem der Ansprüche 22 oder 23, **dadurch gekennzeichnet, dass** der Riegel (77) an einem den Arbeitshub des bewegbaren Teils (13) begrenzenden Bereich vorgesehen ist.

25. Chirurgisches Instrument nach einem der Ansprüche 22 bis 24, **dadurch gekennzeichnet, dass** der Riegel (77) durch einen Druck-, Zug-Schiebemechanismus lösbar ist.

## Claims

1. Surgical instrument with a main part (12) and at least one part (13) movable in guides (31, 32) relative thereto, with a handle (14) arranged on the main part (12) and having a stationary handle portion (16) and an actuatable handle portion (19) which actuates the movable part (13), with a locking device (25, 56, 66, 76) in which in a first position (33) the movable part (13) is arranged in an initial position (22) and in a second position (34) the movable part (13) is changed over into a cleaning position (46) and the movable part (13) is in the cleaning position (46) arranged captive with respect to the main part (12), **characterized in that** the movable part (13) on changing over from the initial position (22) into the cleaning position (46), gets free from a guide (31, 32) and is at least partially pivotable around a pivot axis of an articulated connection (41) between the actuatable handle portion (19) and the movable part (13).

2. Surgical instrument according to claim 1, wherein the movable part (13) and the actuatable handle portion (19) are arranged pivotably with respect to each other by means of a joint-like, slide-like, or hinge-like connection (41).

3. Surgical instrument according to one of the proceeding claims, **characterized in that** upon changing over the moved part (13) from a cleaning position (46) to the working position (22), the guide sections (31) of the movable part engage in seatings of the main part (12), and by means of pressing the actuatable handle portion (19) the guides (31, 32) automatically engage in each other.

4. Surgical instrument according to claim 3, **characterized in that** the guide (32) in the main part (12) has a first section which runs obliquely, and which moves the movable part (13) toward the main part (12) and changes it over into a working position (22).

5. Surgical instrument according to one of the proceeding claims, **characterized in that** the locking device (26, 56, 67, 77) has a latch (27, 57, 67, 77) which can be frictionally and/or positively disposed at least in a locking position (33).

6. Surgical instrument according to one of the proceeding claims, **characterized in that** the locking device (26, 56, 66) has a latch (27, 57, 67) which is provided on the handle (14).

7. Device according to claim 6, wherein the latch (27, 57, 67) is provided on the stationary or actuatable handle portion (16, 19).

8. Surgical instrument according to claim 7, wherein a pivotable latch (27) is provided on the stationary handle portion (19) which engages the actuatable handle portion (19) near a hinge pin (18).

9. Surgical instrument according to claim 8, wherein the pivotable latch (27) has a bounding element (47) determining its locking position (33).

10. Surgical instrument according to one of the claims 5 to 9, **characterized in that** the latch (27) in an unlocking position (34) pivotably releases a further pivoting region of the actuatable handle portion (19), which is limited by a stop (37) provided on the main part (12).

11. Surgical instrument according to one of the claims 5 to 10, **characterized in that** the latch (27) engages on a lever section (28) between the hinge pin (18) and the articulation (41).

12. Surgical instrument according to one of the claims 5 to 11, **characterized in that** the latch (27) has a locking section which engages on a complementarily constituted lever section (28) of the actuatable handle portion (19).

13. Surgical instrument according to claim 12, **characterized in that** the locking section has an undercut, which is preferably formed by a bevel and a thereto adjoining detent cam.

14. Surgical instrument according to one of the claims 1 to 7, **characterized in that** the latch (57) engages a section of the actuatable handle portion (19) opposite the lever section (28).

15. Surgical instrument according to claim 14, wherein the latch (57) is guided in a recess (58) of the actuatable handle position (19).

16. Surgical instrument according to one of the claims 14 or 15, **characterized in that** a shoulder (59) is provided on the latch (57) and positions the actuatable handle portion (19) in an initial position (22).

17. Surgical instrument according to one of the claims 14 to 16, **characterized in that** by releasing the latch (57) from an edge region of the recess (58), a further pivoting region of the actuatable handle portion (19) is released and the actuatable handle portion (19) is pivotable as far as the stop (37) on the main part (12) or a further stop of the latch (57).

18. Surgical instrument according to one of the claims 14 to 17, **characterized in that** on changing over the actuatable handle portion (19) from a cleaning position (46) into a working position (22) an automatic locking of the latch (57) is provided and preferably the actuatable handle portion (19) is arranged in an initial position.

19. Surgical instrument according to one of the claims 1 to 7, **characterized in that** a latch (67) is provided on the stationary handle portion (16) and engages on the movable part (13), preferably at an end section of the movable part (13).

20. Surgical instrument according to claim 19, **characterized in that** the latch (67) is constituted pivotably or displaceably.

21. Surgical instrument according to one of the claims 19 or 20, **characterized in that** the latch (67) is secured in a locking position (33), preferably by means of a releasable detent connection.

22. Surgical instrument according to one of the claims 1 to 7, **characterized in that** the locking device (67) has a latch (77) which is provided on the main part (12) or on the movable part (13).

23. Surgical instrument according to claim 22, **characterized in that** the locking device (76) provided on the main part (12) has a latch (77) which engages in a guide (32) in the main part (12), in which guide the movable part (13) is guided at least sectionwise.

24. Surgical instrument according to one of the claims 22or 23, **characterized in that** the latch (77) is provided on a region limiting the working stroke of the movable part (13).

25. Surgical instrument according to one of the claims 22 to 24, **characterized in that** the latch (77) is releasable by means of a pressing, pulling, or sliding mechanism.

## Revendications

1. Instrument chirurgical comprenant une partie principale (12) et au moins une partie (13) mobile par rapport à celle-ci dans une glissière (31, 32), une poignée (14) disposée sur la partie principale (12), qui présente une portion de poignée fixe (16) et une portion de poignée (19) pouvant actionner la partie mobile (13), un dispositif de verrouillage (26, 56, 66, 76) dans lequel, dans une première position (33), la partie mobile (13) est disposée dans une position initiale (22) et dans une deuxième position (34), la partie mobile (13) est transférée dans une position de nettoyage (46) et la partie mobile (13) est disposée dans cette position de nettoyage (46) de manière imperdable par rapport à la partie principale (12), **caractérisé en ce que** la partie mobile (13), lors du transfert de la position initiale (22) dans la position de nettoyage (46) sort de la glissière (31, 32) et est pivotée au moins partiellement autour d'un axe de rotation d'une connexion articulée (41) formée entre la portion de poignée (19) actionnable et la partie mobile (13).

2. Instrument chirurgical selon la revendication 1, **caractérisé en ce que** la partie mobile (13) et la portion de poignée actionnable (19) sont disposées de manière à pouvoir pivoter l'une par rapport à l'autre par une connexion articulée (41) de type articulation, coulisse ou charnière.

3. Instrument chirurgical selon l'une quelconque des revendications précédentes, **caractérisé en ce que** lors du transfert de la partie mobile (13) d'une position de nettoyage (46) dans la position de travail (22), les portions de glissière (31) de la partie mobile viennent en prise dans des logements de la partie principale (12) et par pression de la portion de poignée actionnable (19), les glissières (31, 32) viennent en prise automatiquement l'une dans l'autre.

4. Instrument chirurgical selon la revendication 3, **caractérisé en ce que** la glissière (32) présente, dans la partie principale (12), une première portion s'étendant obliquement, qui déplace la partie mobile (13) vers la partie principale (12) et la transfère dans une position de travail (22).

5. Instrument chirurgical selon l'une quelconque des revendications précédentes, **caractérisé en ce que** le dispositif de verrouillage (26, 56, 67, 77) présente un verrou (27, 57, 67, 77) est disposé par engagement par force et/ou par coopération de forme au moins dans une position de verrouillage (33).

6. Instrument chirurgical selon l'une quelconque des revendications précédentes, **caractérisé en ce que** le dispositif de verrouillage (26, 56, 66) présente un verrou (27, 57, 67) qui est prévu sur la poignée (14).

7. Instrument chirurgical selon la revendication 6, **caractérisé en ce que** le verrou (27, 57, 67) est prévu sur la portion de poignée fixe ou actionnable (16, 19).

8. Instrument chirurgical selon la revendication 7, **caractérisé en ce qu'**un verrou pivotant (27) est prévu sur la portion de poignée fixe (19), lequel vient en prise à proximité d'un axe de rotation (18) avec la portion de poignée actionnable (19).

9. Instrument chirurgical selon la revendication 8, **caractérisé en ce que** le verrou pivotant (27) présente un élément de limitation (47) déterminant sa position de verrouillage (33).

10. Instrument chirurgical selon l'une quelconque des revendications 5 à 9, **caractérisé en ce que** le verrou (27), dans une position de déverrouillage (34), libère par pivotement une autre région de pivotement de la portion de poignée actionnable (19), qui est limitée par une butée (37) prévue sur la partie principale (12).

11. Instrument chirurgical selon l'une quelconque des revendications 5 à 10, **caractérisé en ce que** le verrou (27) vient en prise sur une portion de levier (28) entre l'axe de pivotement (18) et l'articulation (41).

12. Instrument chirurgical selon l'une quelconque des revendications 5 à 11, **caractérisé en ce que** le verrou (27) présente une portion de verrouillage qui vient en prise avec une portion de levier (28) de la portion de poignée actionnable (19) réalisée de manière complémentaire.

13. Instrument chirurgical selon la revendication 12, **caractérisé en ce que** la portion de verrouillage présente une contre-dépouille qui est réalisée de préférence par un biseau et une saillie d'emboîtement s'y raccordant.

14. Instrument chirurgical selon l'une quelconque des revendications 1 à 7, **caractérisé en ce que** le verrou (57) vient en prise avec une portion de levier (28) qui est en regard de la portion de poignée actionnable (19).

15. Instrument chirurgical selon la revendication 14, **caractérisé en ce que** le verrou (57) est guidé dans un évidement (58) de la portion de poignée actionnable (19).

16. Instrument chirurgical selon l'une quelconque des revendications 14 ou 15, **caractérisé en ce qu'**un épaulement (59) est prévu sur le verrou (57), lequel positionne la portion de poignée actionnable (19) dans une position initiale (22).

17. Instrument chirurgical selon l'une quelconque des revendications 14 à 16, **caractérisé en ce qu'**en libérant le verrou (57) d'une région du bord de l'évidement (58), une région de pivotement supplémentaire de la portion de poignée actionnable (19) est libérée et la portion de poignée actionnable (19) est pivotée jusqu'à la butée (37) sur la partie principale (12) ou un épaulement supplémentaire du verrou (57).

18. Instrument chirurgical selon l'une quelconque des revendications 14 à 17, **caractérisé en ce que** lors du transfert de la portion de poignée actionnable (19) d'une position de nettoyage (46) dans une position de travail (22), un verrouillage automatique du verrou (57) est prévu et la portion de poignée actionnable (19) est de préférence disposée dans une position initiale.

19. Instrument chirurgical selon l'une quelconque des revendications 1 à 7, **caractérisé en ce que** l'on prévoit sur la portion de poignée fixe (16) un verrou (67) qui vient en prise avec la partie mobile (13), de préférence avec une portion d'extrémité de la partie mobile (13).

20. Instrument chirurgical selon la revendication 19, **caractérisé en ce que** le verrou (67) est réalisé de manière à pouvoir pivoter ou coulisser.

21. Instrument chirurgical selon l'une quelconque des revendications 19 ou 20, **caractérisé en ce que** le verrou (67) est fixé dans une position de verrouillage (33), de préférence par une connexion par encliquetage desserrable.

22. Instrument chirurgical selon l'une quelconque des revendications 1 à 7, **caractérisé en ce que** le dispositif de verrouillage (67) présente un verrou (77) qui est prévu sur la partie principale (12) ou la partie mobile (13).

23. Instrument chirurgical selon la revendication 22, **caractérisé en ce que** le dispositif de verrouillage (76) prévu sur la partie principale (12) présente un verrou (77) qui vient en prise dans une glissière (32) dans la partie principale (12), dans laquelle la partie mobile (13) est guidée au moins en partie.

24. Instrument chirurgical selon l'une quelconque des revendications 22 ou 23, **caractérisé en ce que** le verrou (77) est prévu sur une région limitant la course de travail de la partie mobile (13).

25. Instrument chirurgical selon l'une quelconque des revendications 22 à 24, **caractérisé en ce que** le verrou (77) peut être desserré par un mécanisme de tiroir de pression ou de traction.
